# DEMANDE DE BREVET EUROPEEN

(11) **EP 4 534 684 A1**
(43) Date de publication de la demande: **09.04.2025**
(21) Numéro de dépôt: 23201351.6
(22) Date de dépôt: 03.10.2023
(51) Int. Cl.: C12Q 1/04, C12Q 1/25

(54) **DETECTION ET IDENTIFICATION DE LEVURES DU GENRE CANDIDA, METHODES ET OUTILS DE MICROBIOLOGIE POUR CE FAIRE**

(71) Demandeur: BIOMÉRIEUX, 69280 Marcy L'Etoile (FR)
(72) Inventeur: Chansigaud, Gwladys, 69730 Genay (FR); Devigne, Laurence, 01150 Vaux en Bugey (FR); Orenga, Sylvain, 01160 Neuville sur Ain (FR); Roche, Jean Marc, 73350 Feissons sur Salins (FR)
(74) Mandataire: bioMérieux PI Groupement mandataires

(57) **Abrégé**

L'invention a trait aux méthodes de microbiologie et aux milieux de culture dédiés à la détection et à l'identification non-dénaturantes des levures du genre *Candida,* en particulier *C*. *auris, C. albicans, C. glabrata, C. tropicalis, C. parapsilosis* et *C*. *krusei.*

Plus particulièrement, elle porte sur un procédé d'identification de levures du genre *Candida* éventuellement présentes dans un échantillon biologique, comprenant les étapes suivantes :
- mise en culture des levures à identifier sur ou dans un milieu de culture comprenant :
- une composante nutritive apte à permettre le développement et la croissance de levures du genre *Candida,* et
- un inducteur d'a-glucosidase et éventuellement un répresseur d'α-glucosidase,
- un inducteur de phosphatase acide et un répresseur de phosphatase,
- un inducteur de N-acétyl-β-glucosaminidase, et

- détection d'éventuelles activités α-glucosidase, phosphatase acide et/ou N-acétyl-β-glucosaminidase exprimées par les levures, et
- identification de l'espèce à laquelle lesdites levures appartiennent selon les activités α-glucosidase, phosphatase acide et/ou N-acétyl-β-glucosaminidase effectivement exprimées par lesdites levures.

## Description

La présente invention concerne la détection et à l'identification de levures du genre *Candida* par des méthodes et outils de microbiologie analytique.

Plus précisément, elle a trait aux méthodes de microbiologie et aux milieux de culture dédiés à la détection et à l'identification non-dénaturantes de microorganismes, en particulier des levures du genre *Candida,* éventuellement présents dans un échantillon biologique à analyser. Une fois détectés/identifiés avec de tels méthodes de microbiologie et milieux de culture, les microorganismes encore viables pourront optionnellement être récupérés/collectés, multipliés et/ou conservés à des fins d'analyses complémentaires (dénaturantes ou non).

Les levures du genre *Candida* (ou « *Candida spp* ») sont des microorganismes ubiquitaires, largement disséminés dans notre environnement. Ces levures se retrouvent également chez les animaux et les êtres humains avec qui ils entretiennent des relations de commensalisme, en peuplant la peau, la bouche, le canal auditif, les voies respiratoires, le système digestif, les voies génitales...

Chez les sujets à risque (en particulier, les patients immunodéprimés, les diabétiques, les patients sous traitement antibiotique à large spectre et corticoïdes, les patients avec une insuffisance rénale, un stress chronique, les nourrissons, les personnes âgées, les femmes enceintes...), les *Candida spp* peuvent se révéler être des opportunistes hautement pathogènes. Leur prolifération massive et incontrôlée conduit à des infections appelées candidoses, qui peuvent être particulièrement graves, voire létales.

Lorsqu'elles touchent seulement les muqueuses et/ou la peau, ces infections sont qualifiées de « superficielles » et sont généralement bénignes. Dans les formes les plus inquiétantes, les infections atteignent des organes profonds (on parle de « candidoses viscérales ») et/ou se propagent à travers l'organisme (on parle alors de « candidoses invasives » ou de « candidémie »).

Sur le plan sanitaire, les levures du genre *Candida* sont régulièrement identifiées comme la cause d'infections nosocomiales, impliquant bien souvent une contamination du matériel et des outils médicaux utilisés dans les procédures invasives (cathéters, sondes urinaires...). Elles seraient parmi les premières causes de septicémie à l'hôpital qui, dans 40 % des cas, s'avèrent fatales à 30 jours.

Si près de 400 *Candida spp* ont été décrites à ce jour, seule une très faible minorité est responsable de pathologies humaines.

En 2020, le CDC (*Control Disease Center,* US) indiquait qu'au niveau mondial, 39 % des infections invasives liées aux *Candida spp* dans le monde, étaient imputées à *C. albicans,* 28 % à *C*. *glabrata* (aussi désigné par *Nakaseomyces glabrata,* selon une réforme de la nomenclature récemment proposée (Kidd et al., 2023 ; Open Forum Infectious Diseases (2023) : « Fungal Nomenclature: Managing Change is the Name of the Game »)) et 15 % à *C*. *parapsilosis.*

Au niveau de la France, La Société Française de Mycologie (SFM) publiait en 2015 (Référentiel en microbiologie Clinique, Candidose, 5ème édition 2015) qu'environ 50 % des candidoses (c'est-à-dire des infections liées à des levures du genre *Candida*) constatées en France, sont imputables à *C. albicans.* Viennent ensuite, *C. glabrata* (10-20 %), *C. parapsilosis* (10-20 %), *C. tropicalis* (10 %) et *C. krusei* (3 % ; *C*. *krusei,* aussi désigné par *Pichia kudriavzevii,* selon la réforme de nomenclature récemment proposée).

Les autres *Candida spp* sont moins répandues et leur émergence dépend souvent d'écologies particulières.

S'agissant du cas particulier de *C*. *auris,* cette espèce apparue au Japon en 2009, a depuis été surnommée « le Champignon tueur ». Outre sa grande dangerosité, trois autres raisons justifient l'attention et l'inquiétude grandissantes qu'elle suscite actuellement :
- en premier lieu, sa capacité à se propager rapidement dans les établissements de santé où elle se transmet facilement entre les patients, le personnel de santé et les visiteurs ; dans l'environnement hospitalier (en particulier sur les surfaces des équipements hospitaliers, tels que chariots de soins, pompes à perfusion, thermomètres, tensiomètres), *C*. *auris* peut survivre pendant des semaines,
- également, sa multirésistance aux antifongiques (par exemple, au fluconazole, à l'itraconazole, au voriconazole ou encore à l'amphotéricine B), bien que très variable d'une clade géographique à l'autre, limite considérablement les options thérapeutiques disponibles ; de même, sur le plan sanitaire, les mesures de désinfection de l'environnement et du matériel médical, par les ammoniums quaternaires classiques, se sont avérées que peu efficaces,
- enfin, encore peu d'outils analytiques et de diagnostic sont actuellement disponibles pour son identification.

En raison de leur prévalence et de leur dangerosité, les six espèces de levure sus-évoquées (à savoir, *C. auris, C. albicans, C. glabrata, C. tropicalis, C. parapsilosis* et *C. krusei*), sont qualifiées ici de « *Candida spp* majeures » (ou de *« Candida spp* d'intérêt majeur »).

Parce qu'elles infectent les mêmes types de tissus et organes (muqueuses, peau, appareil digestif, appareil respiratoire, organes génitaux...) et donnent lieu à des symptômes très similaires, voire identiques, un simple examen clinique du patient ne permet pas d'identifier précisément et de façon certaine, laquelle de ces six *Candida spp* majeures est effectivement responsable d'un cas particulier de candidose. De plus, compte tenu des résistances aux antifongiques que chacune a développées, des résistances très espèce-dépendantes, il n'y a pas d'approche thérapeutique universelle/commune au traitement des candidoses. Aucun traitement unique ne peut ainsi être systématiquement prescrit en cas de candidose. De ce fait et en vue d'assister les praticiens dans le choix d'un traitement thérapeutique efficace, adapté à chaque patient souffrant de candidose, il est important de pouvoir disposer de méthodes et d'outils aptes à permettre une identification spécifique de chaque *Candida sp* majeure.

La détection et l'identification spécifiques des espèces de levure du genre *Candida* revêtent également une importance capitale sur le plan sanitaire, en particulier dans le cadre de la lutte contre les maladies nosocomiales où l'apparition de tout microorganisme à la surface des outils et instruments chirurgicaux, ou dans les liquides et solutions à usages médicales, est étroitement surveillée.

De nombreuses techniques analytiques permettent déjà une détection et/ou une identification d'espèces de levure du genre *Candida.* Certaines sont basées sur des technologies d'amplification par PCR de séquences nucléiques spécifiques, d'immuno-détection d'antigènes, de spectrométrie de masse. La présente invention s'intéresse plutôt aux méthodes et outils de microbiologie analytique qui ont l'avantage de ne pas dénaturer les levures étudiées, si bien qu'une fois l'identification réalisée, des levures encore viables peuvent être récupérées et possiblement soumises à un antifongigramme en vue d'établir un traitement thérapeutique efficace, adapté au patient infecté. Eventuellement, les spécimens isolés pourront aussi être récupérés à des fins de conservation et/ou d'analyses et études ultérieures.

Plus précisément encore, la présente invention a trait aux méthodes de microbiologie analytique qui mettent en oeuvre des milieux de culture microbiologique solides, sélectifs et discriminants, aptes à permettre une identification/différenciation des *Candida spp,* les unes par rapport aux autres.

Parmi les milieux de culture microbiologique solides, sélectifs et discriminants, connus de l'état de la technique comme destinés à la détection et/ou à l'identification de levures du genre *Candida,* on peut citer notamment les milieux chromogéniques ci-après :
- le CHROMID^{®} Candida agar, développé par la société bioMérieux ; ce milieu de culture solide, qui permet non seulement la croissance des levures en général et particulièrement de *C*. *albicans, C. tropicalis* et *C*. *krusei,* permet aussi l'identification et la différenciation de ces trois espèces de levure grâce à un marquage colorimétrique particulier ; ce milieu ne permet cependant pas une détection/identification spécifique de *C*. *auris,* de *C. glabrata* ou encore de *C. parapsilosis ;*
- les milieux CHROMagar^{™} *Candida* (CHROMagar Microbiology, France), CandiSelect^{™} 4 (BioRad, US) et HiCrome^{™} *Candida differential Agar Base* (HiMedia Laboratories, Inde) permettent de détecter, d'identifier/différencier *C*. *albicans, C. glabrata, C. tropicalis* et *C*. *krusei* ; ils ne permettent cependant pas de détecter/identifier ni *C. auris* ni *C. parapsilosis,* enfin
- le milieu CHROMagar^{™} *Candida* Plus (CHROMagar Microbiology, France) permet une détection de *C*. *auris* ainsi que d'autres *Candida spp* ; mais leur détection souffre d'une faible spécificité (de Jong et al., 2021 ; Journal of Clinical Microbiology (2021), 59(4): « Performance of Two Novel Chromogenic Media for the Identification of Multidrug-Resistant Candida auris Compared with Other Commercially Available Formulations *»).*

Ainsi, à la connaissance de la Demanderesse, il n'existe à ce jour aucune méthode ni outil de microbiologie permettant une détection et une identification simples et satisfaisantes des levures du genre *Candida.* Plus particulièrement, pour la Demanderesse, il n'y a pas à ce jour d'outil qui soit à la fois adapté à la culture de *Candida spp,* quelle qu'en soit l'espèce, et qui permet une discrimination satisfaisante des levures appartenant aux six *Candida spp* majeures que sont *C. auris, C. albicans, C. glabrata, C. tropicalis, C. parapsilosis* et *C*. *krusei.* A la connaissance de la Demanderesse, il n'existe pas non plus ni de méthode ni d'outil de microbiologie qui soient aptes à permettre une détection et une identification suffisamment spécifiques de *C*. *auris.*

Dans ce contexte et pour pallier les déficiences constatées, la présente invention vise à proposer des milieux de culture solides sur lesquels il sera possible de faire pousser différentes *Candida spp* et de les identifier. En particulier, les milieux de culture solide proposés devront non seulement être adaptés à la culture des différentes espèces de levure du genre *Candida,* en particulier *C. auris, C. albicans, C. glabrata* (*Nakaseomyces glabrata*)*, C. tropicalis, C. parapsilosis* et *C*. *krusei* (*P. kudriavzevii*)*,* ils devront également permettre de différencier et d'identifier spécifiquement chacune de ces espèces.

La présente invention porte ainsi sur un milieu de culture microbiologique solide, destiné à l'identification et/ou la discrimination de levures du genre *Candida* (y compris *C*. *glabrata* et *C*. *krusei*)*,* susceptibles d'être présentes dans un échantillon biologique à tester. Pour ce faire, ledit milieu comprend :
- une composante nutritive apte à permettre le développement et la croissance de levures du genre *Candida,* et
- une composante discriminante apte à permettre un marquage visuel des colonies de levures poussant sur ledit milieu de culture, et
est caractérisé en ce que ladite composante discriminante comprend :
- un inducteur d'α-glucosidase, un substrat chromogène et/ou fluorogène d'a-glucosidase qui, sous l'action d'une α-glucosidase, libère un chromophore et/ou un fluorophore émettant un premier signal optique détectable, ainsi qu'éventuellement un répresseur d'a-glucosidase,
- un inducteur de phosphatase acide, un répresseur de phosphatase, ainsi qu'un substrat chromogène et/ou fluorogène de phosphatase qui, sous l'action d'une phosphatase, libère un chromophore et/ou un fluorophore émettant un deuxième signal optique détectable, ledit deuxième signal optique détectable étant différent dudit premier signal optique détectable,
- un inducteur de N-acétyl-β-glucosaminidase, ainsi qu'un substrat chromogène et/ou fluorogène de N-acétyl-β-glucosaminidase qui, sous l'action d'une N-acétyl-β-glucosaminidase, libère un chromophore et/ou un fluorophore émettant un troisième signal optique détectable, ledit troisième signal optique détectable étant différent dudit premier signal optique détectable et dudit deuxième signal optique détectable.

Avant d'aller plus loin dans la présentation et l'explication de l'invention, les définitions suivantes sont données pour en permettre une meilleure compréhension.

Par « échantillon biologique », on entend un échantillon clinique issu d'un prélèvement d'origine humaine ou animale, ou un échantillon alimentaire issu de tout type d'aliment, ou encore un échantillon prélevé dans un environnement particulier (par exemple, un milieu hospitalier, un milieu industriel, un milieu naturel...) par exemple, à des fins de contrôle sanitaire.

Cet échantillon biologique, liquide ou solide, peut être par exemple et de manière non limitative, un échantillon clinique de sang, de plasma, d'urine ou de fèces, un prélèvement de nez, de gorge, de peau, de plaie, de liquide broncho-alvéolaire ou d'expectoration, un échantillon alimentaire tels que le lait, un jus de fruit, un échantillon de yaourt, de viande, d'oeuf, de légume, de mayonnaise, de fromage, de poisson..., un échantillon alimentaire issu d'une alimentation destinée aux animaux, tel que notamment un échantillon de farines animales. A des fins de simplification de vocabulaire, on utilisera indifféremment les expressions « échantillon biologique » et « prélèvement biologique ».

Par « milieu de culture microbiologique solide » ou plus simplement « milieu de culture solide », on entend un support solide ou semi-solide, notamment du type gélosé, gélifié, classiquement utilisé en microbiologie analytique pour cultiver, isoler et/ou identifier des microorganismes. Par opposition aux milieux de culture liquides, un milieu de culture solide incorpore dans sa composition un agent gélifiant, structurant qui lui confère une consistance solide, texturée. A titre d'agent gélifiant/structurant des milieux de culture solides, l'agar (ou agar agar) est largement employé ; des alternatives existent, comme la gélatine, l'agarose, la gomme de guar, la gomme de xanthane...

Un milieu de culture, qu'il soit solide ou liquide, se compose principalement d'une base aqueuse (qui, dans le cas d'un milieu de culture solide, est structurée grâce à un agent gélifiant) tamponnée à un pH approprié, et d'une composante dite nutritive, qui assure l'apport nutritionnel dont les microorganismes ciblés ont besoin pour leur développement et croissance. Très schématiquement, une composante nutritive de milieu de culture renferme classiquement :
- une source carbonée et d'énergie (généralement un glucide, sous la forme d'un sucre simple ou un sucre complexe) ;
- une source de calcium (par exemple CaCl₂.2H₂O) ;
- une source d'azote (par exemple (NH₄)₂SO₄) ;
- une source de soufre (par exemple (NH₄)₂SO₄) ;
- une source de magnésium (par exemple MgCl₂.7H₂O) ;
Une composante nutritive de milieu de culture peut aussi inclure d'autres éléments nutritifs (notamment, acides aminés, facteurs de croissance, vitamines, minéraux, oligo-éléments, citrate de fer, citrate de sodium, chlorure de sodium...) permettant de renforcer les attributs de la composante nutritive. L'apport de la composante nutritive à la composition d'un milieu de culture peut être réalisé pour tout ou parti, au moyen de composés isolés chimiquement bien identifiés, et/ou de compositions dont la formulation est parfaitement définie, et/ou de compositions chimiquement complexes (par exemple, sang frais, sérums, extraits de levure, oeuf entier, jaune d'oeuf, peptones...).

Nombre de milieux de culture sont listés et décrits dans le manuel « *Handbook of Microbiological Media* - *Fourth Edition* » comme étant propices à la culture des levures du genre *Candida.* Le détail de ces milieux de culture, en termes de composition (en particulier, de composante nutritive) et de préparation, tel qu'on le trouve dans ce manuel, est à considérer comme faisant partie intégrante de la présente description. De manière non exhaustive, pour la mise en oeuvre de la présente invention, les composantes nutritives des milieux suivants peuvent être particulièrement avantageuses : les milieux ABY (« *ABYagar - Acid Bismuth Yeast Agar* »), BiGGY (« *BiGGY agar (Bismuth Sulfite Glucose Glycerin Yeast Extract Agar*) »), Candida BCG (« *Candida BCG Agar Base* - *Candida Bromcresol Green Agar Base* »), CHROMagar^{™} Candida Agar (CHROMagar Microbiology, France), CandiSelect^{™} 4 (BioRad, US), *Brillance^{™} Candida Agar* (Thermo Fisher Scientific, US) et *HiCrome^{™} Candida differential Agar Base* (HiMedia Laboratories, Inde).

Par ailleurs, en complément à la composante nutritive, les milieux de culture solides peuvent également intégrer dans leur composition une composante dite discriminante, qui rassemble des composés/ingrédients participant aux processus et mécanismes de détection et d'identification des microorganismes cibles/recherchés. Cela est le cas pour les milieux de culture solides selon l'invention. A cet égard, il convient de noter que la composante discriminante particulière des milieux de culture solides selon l'invention constitue le coeur-même de la présente invention ; elle rend possible la détection et l'identification spécifiques des levures appartenant aux six *Candida spp* majeures que sont *C. auris, C. albicans, C. glabrata, C. tropicalis, C. parapsilosis* et *C*. *krusei.*

La détection et l'identification spécifiques des levures du genre *Candida* mises en oeuvre par les milieux de culture solides selon l'invention reposent, en partie, sur le constat que les *Candida spp* expriment différents niveaux d'activités de type α-glucosidase, phosphatase (en particulier, phosphatase acide) et N-acétyl-β-glucosaminidase. La manifestation de ces activités enzymatiques peut être avantageusement repérée grâce à l'emploi de substrats chromogènes et/ou fluorogènes spécifiques à ces activités enzymatiques ; l'hydrolyse enzymatique de ces substrats libère des chromophores et/ou fluorophores qui teintent localement/ponctuellement la surface du milieu de culture et la biomasse, aux endroits mêmes où se concentrent les activités enzymatiques ciblées et, de ce fait, réalise un marquage visuel des colonies de *Candida spp* exprimant ces activités cibles.

Si en se basant uniquement sur ces trois activités enzymatiques/métaboliques d'intérêt, il est possible de détecter la plupart des espèces du genre *Candida,* le niveau de spécificité d'une telle détection est toutefois insuffisante pour permettre une identification spécifique de chacune des six *Candida spp* majeures (*C*. *auris, C. albicans, C. glabrata, C. tropicalis, C. parapsilosis* et *C*. *krusei*) et/ou une différenciation des ces dernières les unes par rapport aux autres. C'est en cherchant à résoudre ce problème de spécificité de détection et d'identification que les inventeurs ont constaté que ces activités enzymatiques d'intérêt, α-glucosidase, phosphatase acide et N-acétyl-β-glucosaminidase, présentent une certaine sensibilité à des composés modulateurs d'activité métaboliques, en particulier des inducteurs et/ou des répresseurs. Cette sensibilité des activités enzymatiques, en particulier les activités α-glucosidase, phosphatase acide, à des composés inducteurs ou répresseurs d'activité métabolique, varie d'une *Candida sp.* à une autre. C'est en exploitant ingénieusement :
- l'expression différentielle et espèce-dépendante des activités enzymatiques α-glucosidase, phosphatase acide et N-acétyl-β-glucosaminidase, et
- la modulation différentielle et espèce-dépendante des activités enzymatiques α-glucosidase, phosphatase acide et N-acétyl-β-glucosaminidase, par des composés inducteurs et des composés répresseurs particuliers,
que les inventeurs sont parvenus à mettre au point des outils de microbiologie analytique, d'utilisation simple et rapide, qui permettent non seulement une détection des levures de diverses *Candida spp,* mais également d'identifier/discriminer ces levures en fonction de l'espèce à laquelle elles appartiennent, et notamment *C. auris, C. albicans, C. glabrata, C. tropicalis, C. parapsilosis* et *C*. *krusei.*

Ainsi, essentiellement grâce à sa composante discriminante, un milieu de culture solide selon l'invention permet :
- de créer les conditions appropriées pour induire, chez les levures du genre *Candida,* une expression différentielle particulière de trois activités enzymatiques d'intérêt (α-glucosidase, phosphatase acide et N-acétyl-β-glucosaminidase), générant de ce fait un profil d'activité métabolique particulier (tourné vers ces trois activités enzymatiques d'intérêt), de spécificité élevée à l'égard de chaque *Candida sp.,* et
- de visualiser aisément (par colorimétrie et/ou fluorimétrie) chaque colonie de levures poussant sur le milieu et d'en déduire précisément l'espèce en question, en particulier si celle-ci correspond à *C. auris, C. albicans, C. glabrata, C. tropicalis, C. krusei* ou *C. parapsilosis.*

Pour induire l'activité α-glucosidase de levures du genre *Candida,* plus particulièrement de *C. auris, C. albicans, C. tropicalis* et *C. parapsilosis,* un milieu de culture solide selon l'invention comprend avantageusement, à titre d'inducteur d'α-glucosidase, un sucre choisi parmi le maltose et le raffinose, à une concentration par exemple comprise entre 0,05 g/L et 0,5 g/L, de préférence 0,3 g/L pour le maltose et 0,1 g/L pour le raffinose.

Eventuellement, pour réprimer cette activité α-glucosidase chez les levures du genre *Candida,* le galactose et le xylose, utilisés à une concentration comprise entre 5 et 30 g/L, préférentiellement de l'ordre de 20 g/L, peuvent être avantageusement être utilisés.

Selon l'invention, pour induire une activité phosphatase acide chez les levures du genre *Candida,* celles-ci sont placées dans un environnement acide. A cet égard, un tampon pH permet de stabiliser le pH acide du milieu, avantageusement à un pH compris entre 5,5 et 6,5. Pour renforcer davantage l'induction de l'activité phosphatase acide de ces levures, un milieu de culture selon l'invention renferme avantageusement un ou plusieurs sucres fermentaires, métabolisables par les levures. En métabolisant ces sucres fermentaires, les levures produisent des composés acides qui leur sont directement accessibles et qui participent à induire leur activité phosphatase acide.

De manière avantageuse, le milieu de culture comprend un sucre fermentaire métabolisable par les levures et un tampon pH apte à maintenir le milieu à un pH acide, de préférence compris entre 5,5 et 6,5. Avantageusement, le sucre fermentaire est du glucose, utilisé à une concentration comprise entre 5 et 20 g/L.

Pour réprimer l'activité phosphatase acide, le milieu de culture solide renferme avantageusement un sel inorganique de phosphate qui permet de réprimer l'activité phosphatase acide chez certaines *Candida spp,* en l'occurrence, *C. auris, C. albicans* et *C*. *parapsilosis.* Avantageusement et selon l'invention, du phosphate de potassium (KH₂PO₄) est utilisé à cette fin, de préférence à une concentration comprise entre 0,5 g/L et 3 g/L, de préférence 2 g/L.

Enfin, la composante discriminante d'un milieu de culture solide selon l'invention, comprend un inducteur de N-acétyl-β-glucosaminidase, par exemple un hexosamine, tel que par exemple le N-acétyl-glucosamine. Avantageusement et selon l'invention, le N-acétyl-glucosamine à une concentration comprise entre 0,5 g/L et 2 g/L, de préférence 1 g/L, est utilisé pour induire l'activité N-acétyl-β-glucosaminidase de *Candida spp,* et plus particulièrement de *C*. *albicans* et *C*. *tropicalis.*

Ainsi, dans les conditions de culture instaurées par un milieu de culture selon l'invention et dans lequel, de manière différentielle et espèce-dépendante :
- une activité α-glucosidase des *Candida spp* est induite au moyen d'un sucre tel que le maltose ou le raffinose,
- une activité phosphatase acide des *Candida spp* est induite au moyen du glucose, et est réprimée par le phosphate de potassium, et
- une activité N-acétyl-β-glucosaminidase des *Candida spp* est induite par un hexosamine,
les espèces *C. auris, C. albicans, C. glabrata, C. tropicalis, C. krusei* et *C. parapsilosis* expriment chacune un profil d'activité métabolique particulier (basé sur l'observation des trois activités enzymatiques d'intérêt) et spécifique. En l'occurrence :
- *C*. *auris* exprime uniquement l'activité α-glucosidase,
- *C*. *albicans* exprime seulement deux des trois activités enzymatiques d'intérêt, à savoir α-glucosidase et N-acétyl-β-glucosaminidase,
- *C*. *tropicalis* exprime les trois activités enzymatiques d'intérêt,
- *C*. *glabrata* et *C*. *krusei* expriment, tous deux, uniquement l'activité phosphatase acide mais sont visuellement très reconnaissables l'une de l'autre par la morphologie des colonies qu'elles forment sur les milieux de culture solides, y compris un milieu de culture solide selon l'invention ; *C*. *glabrata* forme des colonies à bords lisses alors que *C*. *krusei* forme des colonies à bords découpés, dentelés, et
- *C. parapsilosis* présente un faible niveau d'activité α-glucosidase (et, éventuellement, un niveau d'activité phosphatase résiduel en cas de répression partielle de cette activité, traduisant l'utilisation d'une quantité retreinte de phosphatase de potassium).

Sur un milieu de culture solide selon l'invention, à chaque colonie isolée est ainsi associée une couleur donnée qui reflète le profil d'activité métabolique de l'espèce qui lui correspond :
- la couleur des colonies de *C*. *auris* est celle du chromophore/fluorophore du substrat chromogène/fluorogène d'α-glucosidase,
- la couleur des colonies de *C*. *glabrata* et *C*. *krusei* est celle du chromophore/fluorophore du substrat chromogène/fluorogène de phosphatase,
- la couleur des colonies de *C*. *albicans* résulte du mélange entre les chromophores/fluorophores des substrats chromogènes/fluorogènes d'a-glucosidase et de N-acétyl-β-glucosaminidase,
- la couleur des colonies de *C*. *tropicalis* résulte du mélange entre les chromophores/fluorophores des substrats chromogènes/fluorogènes d'a-glucosidase, de phosphatase et de N-acétyl-β-glucosaminidase,
- quant aux colonies de *C*. *parapsilosis,* elles apparaissent légèrement teintées par le chromophore/fluorophore du substrat chromogène/fluorogène d'α-glucosidase (et éventuellement avec une très légère teinte reflétant celle du chromophore/fluorophore du substrat chromogène/fluorogène de phosphatase, en cas de répression partielle de cette activité).

Pour mettre en oeuvre la présente invention, nombre de substrats chromogènes et/ou fluorogènes applicables aux milieux de culture solides et permettant un marquage colorimétrique et/ou fluorométrique d'activités enzymatiques telles que α-glucosidase, phosphatase et N-acétyl-β-glucosaminidase, sont disponibles dans le commerce et/ou ont déjà été décrits dans la littérature. Les chromophores/fluorophores (et donc, les substrats chromogènes et/ou fluorogènes) représentatifs des trois activités enzymatiques d'intérêt seront sélectionnés spécialement pour leur capacité à créer toute une palette de couleur et/ou de fluorescence, apte à permettre une discrimination visuelle aisée des profils d'activité métabolique induits selon l'invention.

Selon un mode particulier de réalisation de l'invention avantageux, les milieux de culture solides sont chromogéniques et renferment ainsi des substrats chromogènes d'α-glucosidase, de phosphatase et de N-acétyl-β-glucosaminidase.

Il existe une grande variété de substrats chromogéniques à cet effet, et notamment ceux avec des noyaux chromophores de type indoxyl, coumarine, hydroxyquinoline... et des dérivés, ou encore des substrats de la gamme des Aldol^{®} de la société suisse BIOSYNTH AG.

A titre d'exemple de substrats chromogéniques envisageables pour la préparation d'un milieu de culture solide selon l'invention, il peut être cité :
- pour mettre en évidence une activité α-glucosidase :
   - l'Aldol^{®} 455-α-Glu (la lyse enzymatique libère un chromophore de teinte jaune),
   - l'Aldol^{®} 518-α-Glu (la lyse enzymatique libère un chromophore de teinte rouge),
   - l'Aldol^{®} 484-α-Glu (la lyse enzymatique libère un chromophore de teinte rouge),
   - le 5-bromo-4-chloro-3-indolyl-α-D-glucoside (ou X-α-Glu, et dont la lyse enzymatique libère un chromophore de teinte bleue),
   - le 5-bromo-6-chloro-3-indolyl-α-D-glucosidase (ou Mag-α-Glu ; la lyse enzymatique libère un chromophore de teinte rouge magenta),
   - le 6-chloro-3-indolyl-β-D-glucopyranoside (ou Rose-α-Glu ou Sal-α-Glu ; la lyse enzymatique libère un chromophore de teinte rose saumon),
   - le 5-bromo-4-chloro-3-indolyl-N-méthyl-alpha-D-glucoside (ou GreenA-α-Glu ; la lyse enzymatique libère un chromophore de teinte verte/bleu turquoise),
   - l'Alizarine-α-Glu (la lyse enzymatique libère un chromophore dont la teinte dépend de l'ion métallique avec lequel il est utilisé et auquel il pourra se chélater ; par exemple, ce substrat donne une teinte violette en présence de citrate de fer dans le milieu, rose/saumon en présence de sulfate d'aluminium, et mauve rose, en présence de sulfate de cuivre),
- substrats de phosphatase :
   - le 2-naphthyl-phosphate (la lyse enzymatique libère un chromophore de teinte magenta)
   - le 6-bromo-3-indolyl-phosphate (ou Red-Phos ; la lyse enzymatique libère un chromophore de teinte rose)
   - le 5-bromo-6-chloro-3-indolyl-phosphate (ou Mag-Phos ; la lyse enzymatique libère un chromophore de teinte magenta),
   - le 5-bromo-4-chloro-3-indolyl-phosphate (ou X-Phos ; la lyse enzymatique libère un chromophore de teinte verte/bleu turquoise),
   - l'Aldol^{®} 470-phosphate (la lyse enzymatique libère un chromophore de teinte jaune),
   - l'Aldol^{®} 495-phosphate (la lyse enzymatique libère un chromophore de teinte orange),
   - l'Aldol^{®} 515-phosphate (la lyse enzymatique libère un chromophore de teinte rouge), et
- un substrat de β-N-acétyl-glucosaminidase :
   - le 5-bromo-6-chloro-3-indolyl-β-N-acétyl-glucosaminide (ou Mag-NAG ; la lyse enzymatique libère un chromophore de teinte magenta)
   - le 6-chloro-3-indolyl-β-N-acétyl-glucosaminide (ou Rose-NAG ou Sal-NAG ; la lyse enzymatique libère un chromophore de teinte rose saumon),
   - le 5-bromo-4-chloro-3-indolyl-N-acétyl-glucosaminide (ou X-NAG ; la lyse enzymatique libère un chromophore de teinte bleue).

Le bon fonctionnement de certains substrats chromogéniques, par exemple ceux à noyau indoxyl, peuvent nécessiter la présence d'un sel métallique (tel que par exemple, citrate de fer, sulfate d'aluminium, sulfate de cuivre ou chlorure de manganèse).

Selon un premier mode préféré de réalisation de l'invention, pour améliorer la discrimination visuelle entre les colonies de *C*. *auris* et de *C*. *parapsilosis,* basée sur le niveau d'activité α-glucosidase exprimée par ses deux *Candida spp,* à titre de substrat chromogène, on utilise avantageusement des dérivés d'indoxyl à base d'Aldol^{®}, c'est-à-dire des substrats chromogènes porteurs d'un noyau indoxyl (1H-indolyl-3-yl) conjugué sur l'amine cyclique (N-arylés). La structure et la préparation de tels substrats chromogènes sont décrites dans la demande internationale PCT référencée WO 2010/128120, déposée au nom de Biosynth AG, une compagnie suisse qui commercialise également par elle-même de tels substrats chromogènes.

Avec de tels substrats chromogènes d'a-glucosidase, notamment l'Aldol^{®} 455-α-D-glucopyranoside (CAS No. 1254798-01-0 ; Biosynth réf. A-4689_P00) les colonies de *C*. *auris* revêtent une coloration franche et marquée, alors que les colonies de *C*. *parapsilosis* apparaissent faiblement colorées.

Selon un deuxième mode préféré de réalisation de l'invention, qui peut venir en complément ou alternative au premier mode préféré, pour améliorer la discrimination visuelle entre les colonies de *C*. *auris* et de *C*. *parapsilosis,* l'activité α-glucosidase exprimée chez ces deux espèces de levures, est modulée au moyen d'un répresseur. A ce titre, le galactose ou le xylose, utilisé à une concentration comprise entre 5 et 30 g/L, préférentiellement de l'ordre de 20 g/L, permet de réprimer fortement l'activité α-glucosidase de *C. parapsilosis* et de manière nettement moins notable, celle de *C*. *auris.*

Ainsi, dans les conditions de culture instaurées conformément à ce deuxième mode préféré de réalisation d'un milieu de culture selon l'invention et dans lequel :
- une activité α-glucosidase des *Candida spp* est induite au moyen d'un sucre tel que le maltose ou le raffinose, et réprimée par le galactose ou le xylose,
- une activité phosphatase acide des *Candida spp* est induite au moyen du glucose, et est réprimée par le phosphate de potassium, et
- une activité N-acétyl-β-glucosaminidase des *Candida spp* est induite par un hexosamine,
les espèces *C. auris, C. albicans, C. glabrata, C. tropicalis, C. krusei* et *C. parapsilosis* expriment chacune un profil d'activité métabolique particulier (basé sur l'observation des trois activités enzymatiques d'intérêt) et spécifique. En l'occurrence :
- *C*. *auris* exprime uniquement l'activité α-glucosidase,
- *C*. *albicans* exprime seulement deux des trois activités enzymatiques d'intérêt, à savoir α-glucosidase et N-acétyl-β-glucosaminidase,
- *C*. *tropicalis* exprime les trois activités enzymatiques d'intérêt,
- *C*. *glabrata* et *C*. *krusei* expriment, tous deux, uniquement l'activité phosphatase acide mais sont visuellement très reconnaissables l'une de l'autre par la morphologie des colonies qu'elles forment sur les milieux de culture solides, y compris un milieu de culture solide selon l'invention ; *C*. *glabrata* forme des colonies à bords lisses alors que *C*. *krusei* forme des colonies à bords découpés, dentelés, et
- *C. parapsilosis* n'exprime aucune des trois activités enzymatiques d'intérêt (éventuellement on peut constater une faible activité phosphatase en cas de répression partielle de cette activité due à l'utilisation d'une quantité retreinte de phosphate de potassium).

Sur un milieu de culture solide conforme à ce deuxième mode préféré de réalisation de l'invention, à chaque colonie isolée de *Candida sp* particulière est ainsi associée une couleur donnée qui reflète le profil d'activité métabolique de l'espèce qui lui correspond :
- la couleur des colonies de *C*. *auris* est celle du chromophore/fluorophore du substrat chromogène/fluorogène d'α-glucosidase,
- la couleur des colonies de *C*. *glabrata* et *C*. *krusei* est celle du chromophore/fluorophore du substrat chromogène/fluorogène de phosphatase,
- la couleur des colonies de *C*. *albicans* résulte du mélange entre les chromophores/fluorophores des substrats chromogènes/fluorogènes d'a-glucosidase et de N-acétyl-β-glucosaminidase,
- la couleur des colonies de *C*. *tropicalis* résulte du mélange entre les chromophores/fluorophores des substrats chromogènes/fluorogènes d'a-glucosidase, de phosphatase et de N-acétyl-β-glucosaminidase,
- quant aux colonies de *C*. *parapsilosis,* elles apparaissent incolores (et éventuellement avec une très légère teinte reflétant celle du chromophore/fluorophore du substrat chromogène/fluorogène de phosphatase, en cas de répression partielle de cette activité).

Indépendamment du mode préféré de réalisation de l'invention, de manière avantageuse :
- l'activité d'α-glucosidase est avantageusement associée à un chromophore jaune ou une couleur similaire (par exemple, en utilisant comme substrat chromogène, l'Aldol^{®} 455-α-glucoside),
- l'activité phosphatase est avantageusement associée à un chromophore rose, rouge ou une teinte similaire (par exemple, en utilisant comme substrat chromogène, le Magenta-phosphate, l'Aldol^{®} 484-phosphate, l'Aldol^{®} 518-phosphate, l'alizarine-phosphate, ou le Rose-phosphate...), et
- l'activité N-acétyl-β-glucosaminidase est avantageusement associée à un chromophore bleu ou une couleur similaire (par exemple, en utilisant comme substrat chromogène, le X-N-acétyl-β-glucosaminide).

Avantageusement, un milieu de culture solide selon l'invention est un milieu dit sélectif pour les levures du genre *Candida.* A cet égard, outre la composante nutritive et la composante discriminante, un milieu de culture solide selon l'invention comprend également une composante sélective qui favorise la culture des *Candida spp* plutôt que celle de la flore accompagnatrice éventuellement présente dans les échantillons biologiques à tester. A cet égard, sa composition intègre une composante sélective, qui comprend un ou plusieurs composés ayant un effet inhibiteur ou biocide sur les microorganismes « *non-Candida* ». En l'occurrence, ladite composante sélective comprend un ou plusieurs des antibiotiques suivants : amikacine, vancomycine, ceftazidime, chloramphénicol.

Avantageusement, la composante nutritive d'un milieu de culture selon l'invention comprend une teneur en extrait de levure inférieure à 0,5 g/L. De préférence, elle est exempte d'extrait de levure.

La présente invention s'étend également à un procédé d'identification de levures du genre *Candida* éventuellement présentes dans un échantillon biologique, comprenant les étapes suivantes :
- mise en culture de microorganismes à étudier sur ou dans un milieu de culture comprenant :
   - une composante nutritive apte à permettre le développement et la croissance de levures du genre *Candida,*
   - un inducteur d'α-glucosidase et éventuellement un répresseur d'a-glucosidase,
   - un inducteur de phosphatase acide et un répresseur de phosphatase, et
   - un inducteur de N-acétyl-β-glucosaminidase,
- détection d'éventuelles activités α-glucosidase, phosphatase acide et/ou N-acétyl-β-glucosaminidase exprimées par les levures, et
- identification de l'espèce à laquelle lesdites levures appartiennent selon les activités α-glucosidase, phosphatase acide et/ou N-acétyl-β-glucosaminidase effectivement exprimées par lesdites levures.

La présente invention s'étend aussi à un procédé d'identification de *C*. *auris,* éventuellement présent dans un échantillon biologique, comprenant les étapes suivantes :
- mise en culture de microorganismes à étudier sur ou dans un milieu de culture comprenant :
   - une composante nutritive apte à permettre le développement et la croissance de levures du genre *Candida,*
   - un inducteur d'α-glucosidase et éventuellement un répresseur d'a-glucosidase,
   - un inducteur de phosphatase acide et un répresseur de phosphatase,
   - un inducteur de N-acétyl-β-glucosaminidase,
- détection d'éventuelles activités α-glucosidase, phosphatase acide et/ou N-acétyl-β-glucosaminidase exprimées par les levures, et
- identification comme *C*. *auris,* les levures qui, dans lesdites conditions de culture, expriment une activité α-glucosidase et n'expriment ni une activité phosphatase acide, ni une activité N-acétyl-β-glucosaminidase.

Avantageusement, dans un procédé d'identification de levures du genre *Candida* selon l'invention ainsi que dans un procédé d'identification de *C*. *auris* selon l'invention, lesdites activités α-glucosidase, phosphatase acide et N-acétyl-β-glucosaminidase éventuellement exprimées, sont détectées (ou mises en évidence) au moyen de substrats chromogènes et/ou fluorogènes.

Avantageusement et selon l'invention, ledit procédé d'identification de levures du genre *Candida* et ledit procédé de détection et/ou d'identification *C*. *auris* selon l'invention, se caractérisent également par tout ou partie des caractéristiques techniques suivantes :
- l'inducteur d'α-glucosidase est un sucre choisi parmi le maltose et le raffinose, à une concentration par exemple comprise entre 0,05 g/L et 0,5 g/L, de préférence une concentration de 0,3 g/L pour le maltose et de 0,1 g/L pour le raffinose ;
- optionnellement, l'activité α-glucosidase exprimée chez certaines *Candida spp* est réprimée par le galactose ou le xylose, utilisé à une concentration comprise entre 5 g/L et 30 g/L, notamment de l'ordre de 20 g/L ;
- l'activité phosphatase acide est induite en plaçant les levures dans un environnement acide, tamponné à un pH compris entre 5,5 et 6,5, et en présence d'un ou de plusieurs sucres fermentaires, tels que le glucose à une concentration comprise entre 5 g/L et 20 g/L ;
- la répression de l'activité phosphatase est obtenue au moyen d'un sel inorganique de phosphate, tel que par exemple le phosphate de potassium (KH₂PO₄) qui, utilisé à une concentration comprise entre 0,5 g/L et 3 g/L, de préférence 2 g/L, a démontré un effet répresseur sur l'activité phosphatase chez certaines *Candida spp,* en l'occurrence, *C. auris, C. albicans* et *C. parapsilosis* ; et
- l'induction de l'activité N-acétyl-β-glucosaminidase est obtenue en utilisant un hexosamine, tel que par exemple le N-acétyl-glucosamine, à une concentration comprise entre 0, 5 g/L et 2 g/L, de préférence 1 g/L.

Avantageusement et selon l'invention, ledit procédé d'identification de levures du genre *Candida* selon l'invention et ledit procédé de détection et/ou d'identification *C*. *auris* sont mis en oeuvre au moyen d'un milieu de culture solide tel que précédemment décrit.

D'autres buts, caractéristiques et avantages de l'invention apparaîtront au vu de la description qui va suivre et des exemples développés ci-dessous, qui ont pour but de faciliter la compréhension de l'invention et de sa mise en oeuvre. Ces exemples sont donnés à titre explicatif et ne sauraient limiter la portée de l'invention.

Les exemples ci-après rapportés ne reprennent qu'en partie les travaux menés par les inventeurs dans le cadre du développement et de la conception de la présente invention, et ont trait aux données estimées comme pertinentes pour l'appréciation du mérite de l'invention et de celui des inventeurs.

### EXEMPLES

### 1/ - Evaluation des activités métaboliques α-glucosidase, phosphatase acide et N-acétyl-β-glucosaminidase, exprimées par les six Candida spp majeures

Les tests réalisés dans le cadre de la présente invention et tels qu'exposés ci-après concernent 38 souches pures de levures du genre *Candida,* couvrant six espèces différentes, à savoir :
- 9 souches de *C*. *auris,* provenant des clades I, II, III et IV,
- 7 souches de *C*. *albicans,*
- 5 souches de *C*. *glabrata*
- 6 souches de *C*. *tropicalis,*
- 6 souches de *C*. *krusei,* et
- 5 souches de *C. parapsilosis.*

Ces 38 souches de levure proviennent toutes de la collection privée de bioMérieux. Ces souches ont été collectées au fil des années, auprès de divers organismes tels que :
- le CDC (« *Centersfor Disease Control and Prévention* »), aux Etats-Unis ;
- le CBS (« *Centraal Bureau voor Schimmelcultures* »), aux Pays-Bas ;
- le BCCM (« *Belgian Coordinated Collections of Microorganisms* »), en Belgique ;
- le *Leibniz Institute DSMZ,* en Allemagne
- le CHUV (Centre Hospitalier Universitaire Vaudois), en Suisse ;
- l'Hôpital universitaire de la Charité de Berlin (« *Charité* - *Universitäitsmedizin Berlin* »), en Allemagne ;
- la Faculté de Pharmacie de Montpellier, en France ;
- le Centre Hospitalier Bellevue de Saint-Etienne, en France ;
- l'Hôpital Michallon de la Tronche, en France ; et
- le Centre Hospitalier Universitaire de Lille, France.

Ces six espèces de levure ont été évaluées pour leur capacité à exprimer différentes activités métaboliques dans des conditions particulières, établies sur un milieu de culture solide ayant comme composition de base celle d'un milieu SDA (« *Sabouraud Dextrose Agar* »)*.*

Il convient de noter qu'avant de pouvoir retenir les trois activités métaboliques que sont l'α-glucosidase, la phosphatase acide et la N-acétyl-β-glucosaminidase, comme activités enzymatiques d'intérêt pour la détection et l'identification des levures du genre *Candida,* les inventeurs ont envisagé et étudié près de quatre-vingts activités enzymatiques parmi lesquelles des osidases (α-glucosidase, β-glucosidase, α-maltosidase, ribosidase...), des phosphatases, des estérases, des sulfatases, des phospholipases, des peptidases et des nitro-réductases. Au final, la combinaison entre α-glucosidase, phosphatase acide et N-acétyl-β-glucosaminidase a été retenue comme possédant un potentiel prometteur pour une bonne différenciation/discrimination des espèces du genre *Candida,* et une bonne caractérisation de *C*. *auris.*

Seuls sont retracés ci-après, les travaux en lien étroit avec les activités α-glucosidase, phosphatase acide et N-acétyl-β-glucosaminidase, et ceux visant à mettre en évidence un éventuel impact de modulateurs d'activité métabolique (en particulier, le galactose, le xylose, le phosphate de potassium) sur ces trois activités métaboliques.

### 1.1/ - Activité α-glucosidase et répression optionnelle

Parmi les activités métaboliques possiblement exprimées par les *Candida spp,* les inventeurs se sont intéressés à l'activité α-glucosidase.

Il a ainsi pu être constaté qu'une activité α-glucosidase était effectivement exprimée chez *C. auris, C. albicans, C. tropicalis* et *C. parapsilosis.* A l'inverse, aucune activité α-glucosidase n'a pu être détectée chez *C*. *glabrata* et *C*. *krusei.*

En vue d'optimiser davantage le pouvoir discriminant de l'activité α-glucosidase à l'égard des *Candida spp,* différents répresseurs ont été évalués, notamment divers sucres et en particulier, des sucres simples. Un effet répresseur de l'activité α-glucosidase exprimée par *C*. *parapsilosis* a ainsi pu être constaté, aussi bien avec le galactose que le xylose. A l'inverse, ces derniers sont sans effet sur l'activité α-glucosidase de *C*. *auris, C. albicans* et *C. tropicalis.*

Le Tableau 1 ci-après résume très brièvement les résultats des travaux de détection d'une activité α-glucosidase réalisés sur les six *Candida spp* majeures, en présence ou non d'un répresseur (en l'occurrence, galactose ou xylose) de cette activité métabolique particulière. La détection de l'activité α-glucosidase a été réalisée en utilisant le 5-bromo-4-chloro-3-indolyl-alpha-D-glucoside (ou X-α-Glu).

Le signe (+) indique que l'activité enzymatique d'intérêt a effectivement été constatée et repérée par une coloration franche des colonies. Le signe (-) indique que des colonies sont bien apparues sur le milieu de culture mais que celles-ci sont restées incolores ; ceci atteste d'une activité enzymatique d'intérêt non détectée, dans les conditions de répression testées. Le signe (+/-) indique une activité enzymatique d'intérêt moins prononcée et des colonies de coloration moins intense, relativement pâle.

**Tableau 1**

| | α-glucosidase (+maltose) | α-glucosidase (+maltose, galactose) | α-glucosidase (+maltose, xylose) |
|---|---|---|---|
| *C. auris* | + | + | + |
| *C. albicans* | + | + | + |
| *C. glabrata* | - | - | - |
| *C. tropicalis* | + | + | + |
| *C. krusei* | - | - | - |
| *C. parapsilosis* | +/- | - | - |

S'agissant plus spécifiquement du marquage chromogénique de l'activité α-glucosidase des *Candida spp,* des substrats chromogènes de différentes structures ont été testés, notamment en vue de sélectionner les chromophores permettant d'obtenir une bonne différenciation visuelle des colonies de *C*. *auris* et des colonies de *C*. *parapsilosis,* qui toutes expriment une activité α-glucosidase, mais à des niveaux différents.

Le Tableau 2 ci-après rapporte une synthèse de résultats de travaux sur quelques chromogènes testés à cet égard. La coloration et l'intensité constatées sont reportées schématiquement dans ce tableau. Plus la valeur numérique indiquée est élevée, plus intense est la coloration.

**Tableau 2**

| | Aldol^{®} 455-α-Glu (0,1 g/L) | | Mag-α-Glu (0,3 g/L) | | Sal-α-Glu (0,3 g/L) | | Alizarine-α-Glu (0,05 g/L) | |
|---|---|---|---|---|---|---|---|---|
| | +maltose | +maltose +galactose | +maltose | +maltose +galactose | +maltose | +maltose +galactose | +maltose | +maltose +galactose |
| *C. auris* | jaune 1,5 à 3 | jaune 1 à 2 | violet 2à3 | violet 1 à 2 | rose 2à3 | rose 1 à 2 | mauve 1,5 à 2 | mauve 2 |
| *C. parapsilosis* | jaune 0,5 | incolore | violet 1 à 2 | incolore | rose 1,5 à 2 | incolore | rose 1 à 2 | incolore |

Avec les substrats chromogènes dérivés d'indoxyl à base d'Aldol^{®}, tels que l'Aldol^{®} 455-α-Glu, les inventeurs ont obtenu une excellente discrimination visuelle entre les colonies de *C*. *auris* et de *C*. *parapsilosis,* avec ou sans galactose par une différence significative d'intensité de coloration entre les deux espèces.

### 1.2/ - Activité phosphatase acide, et influence du glucose et du KH₂PO₄

L'activité phosphatase acide est une deuxième activité métabolique des levures du genre *Candida* à laquelle les inventeurs se sont intéressés.

Leur travaux entrepris ont conduit notamment aux constats suivants :
- *C. auris, C. albicans, C. glabrata, C. tropicalis, C. krusei* et *C. parapsilosis* expriment tous une activité phosphatase acide ;
- chez *C. auris, C. albicans* et *C. parapsilosis,* cette activité métabolique peut être réprimée par un sel inorganique de phosphate, par exemple le phosphate de potassium (KH₂PO₄), notamment utilisé à une concentration de 0,5 g/L à 3 g/L ; cet effet de répression de l'activité phosphatase n'a cependant pas été constaté chez *C*. *glabrata, C. tropicalis* et *C. krusei.*

Le Tableau 3 ci-après résume très brièvement les résultats des travaux de détection d'une activité phosphatase acide chez les six *Candida spp* majeures, en présence ou non d'un répresseur tel que le phosphate de potassium.

Le signe (+) indique que l'activité enzymatique d'intérêt a effectivement été constatée. La détection d'une activité phosphatase a été réalisée en utilisant le 5-bromo-6-chloro-3-indolyl-phosphate (ou Mag-Phos). Le signe (-) indique que des colonies sont bien apparues sur le milieu de culture mais que celles-ci sont restées incolores ; ceci atteste d'une activité enzymatique d'intérêt non détectée, dans les conditions de répression testées.

**Tableau 3**

| | phosphatase acide (+ glucose) | phosphatase acide (+ glucose, KH₂PO₄) |
|---|---|---|
| *C. auris* | + | - |
| *C. albicans* | + | - |
| *C. glabrata* | + | + |
| *C. tropicalis* | + | + |
| *C. krusei* | + | + |
| *C. parapsilosis* | + | - |

### 1.3/ - Activité N-acétyl-β-glucosaminidase

Enfin, les inventeurs ont sélectionné l'activité N-acétyl-β-glucosaminidase pour son pouvoir discriminant des levures du genre *Candida.* Il a ainsi pu être constaté l'expression de cette activité métabolique chez *C. albicans* et *C. tropicalis.* En revanche, aucune activité N-acétyl-β-glucosaminidase n'a pu être observée chez *C*. *auris, C. parapsilosis, C. glabrata* et *C*. *krusei.*

A cet effet, les levures ont été stimulés par du N-acétyl-β-glucosamine (à une concentration finale dans le milieu de 1 g/L).

Le Tableau 4 ci-après résume très brièvement les résultats des travaux de détection d'une activité N-acétyl-β-glucosaminidase chez les six espèces majeures du genre *Candida.* La détection de l'activité phosphatase a été réalisée en utilisant le 5-bromo-4-chloro-3-indolyl-N-acétyl-glucosaminide (ou X-NAG). Le signe (+) indique que l'activité enzymatique d'intérêt a effectivement été constatée. Le signe (-) indique que des colonies sont bien apparues sur le milieu de culture mais que celles-ci sont restées incolores.

**Tableau 4**

| | N-acétyl-β-glucosaminidase (+N-acétyl-β-glucosamine) |
|---|---|
| *C. auris* | - |
| *C. albicans* | + |
| *C. glabrata* | - |
| *C. tropicalis* | + |
| *C. krusei* | - |
| *C. parapsilosis* | - |

Par ailleurs, les inventeurs ont constaté que le glucose exerce un effet répresseur concentration-dépendant sur l'activité N-acétyl-β-glucosaminidase. Dès lors, dans l'éventualité où, concomitamment à une détection d'activité N-acétyl-β-glucosaminidase, l'utilisation du glucose serait envisagée, par exemple pour stimuler une activité glucosidase (α- et/ou β-glucosidase) et/ou une activité phosphatase acide (dans ce cas, le glucose est utilisé à titre de sucre fermentaire permettant d'établir un environnement acide à proximité immédiate des colonies formées), sa concentration dans le milieu devra être choisie suffisamment faible pour éviter qu'il n'interfère avec l'expression de la N-acétyl-β-glucosaminidase. Une concentration en glucose inférieure ou égale à 20 g/L, de préférence de l'ordre de 5 g/L, a été établie comme appropriée pour éviter cette interférence avec l'activité N-acétyl-β-glucosaminidase de *C*. *albicans* et *C*. *tropicalis,* dans les conditions de mise en oeuvre de la présente invention.

Contrairement au glucose, le galactose ne semble pas avoir d'impact sur l'expression de N-acétyl-β-glucosaminidase chez *C*. *albicans* et *C*. *tropicalis.* Dès lors, de manière concomitant à un marquage de *C. albicans* et/ou *C. tropicalis* par l'activité N-acétyl-β-glucosaminidase, une répression de l'expression l'α-glucosidase chez *C. parapsilosis* peut être opérée sur un même milieu de culture, sans qu'il ne soit nécessaire d'accorder une précaution particulière quant au choix de la concentration de galactose à utiliser pour ne pas bloquer l'activité N-acétyl-β-glucosaminidase.

### 1.4/ - Trois activités métaboliques pour discriminer les six Candida spp majeures.

### 1.4.1/ - Selon le premier mode préféré de réalisation de l'invention

Le Tableau 5 ci-après récapitule très schématiquement et de manière synthétique les résultats présentés plus haut, selon le premier mode de réalisation de l'invention, c'est-à-dire sans répression différentielle de l'activité α-glucosidase et dans lequel un dérivé d'indoxyl à base d'Aldol^{®}, en l'occurrence l'Aldol^{®} 455-α-D-glucopyranoside, est avantageusement utilisé comme substrat chromogène d'a-glucosidase.

Le signe (+) indique que l'activité enzymatique d'intérêt a effectivement été constatée et repérée par une coloration franche des colonies. Le signe (-) indique que des colonies sont bien apparues sur le milieu de culture mais que celles-ci sont restées incolores ; ceci atteste d'une activité enzymatique d'intérêt non détectée, dans les conditions de répression testées. Le signe (+/-) indique une activité enzymatique d'intérêt notablement moins prononcée, reflétée par une coloration significativement moins intense.

**Tableau 5**

| | α-glucosidase (+maltose) | phosphatase acide (+glucose, KH₂PO₄) | N-acétyl-β-glucosaminidase (+N-acétyl-β-glucosamine) | Profil métabolique |
|---|---|---|---|---|
| *C. auris* | + | - | - | profil A |
| *C. albicans* | + | - | + | profil B |
| *C. glabrata* | - | + | - | profil C |
| *C. tropicalis* | + | + | + | profil D |
| *C. krusei* | - | + | - | profil C |
| *C. parapsilosis* | +/- | - | - | profil E |

### 1.4.2/ - Selon le deuxième mode préféré de réalisation de l'invention

Le Tableau 6 ci-après récapitule très schématiquement et de manière synthétique les résultats présentés plus haut, selon le deuxième mode préféré de réalisation de l'invention, c'est-à-dire avec une répression différentielle de l'activité α-glucosidase (au moyen de galactose ou xylose, rajouté au milieu de culture).

Le signe (+) indique que l'activité enzymatique d'intérêt a effectivement été constatée et repérée par une coloration franche des colonies. Le signe (-) indique que des colonies sont bien apparues sur le milieu de culture mais que celles-ci sont restées incolores ; ceci atteste d'une activité enzymatique d'intérêt non détectée, dans les conditions de répression testées.

**Tableau 6**

| | α-glucosidase (+ maltose, galactose/xylose) | phosphatase acide (+ glucose, KH₂PO₄) | N-acétyl-β-glucosaminidase (+ N-acétyl-β-glucosamine) | Profil métabolique |
|---|---|---|---|---|
| *C. auris* | + | - | - | profil A |
| *C. albicans* | + | - | + | profil B |
| *C. glabrata* | - | + | - | profil C |
| *C. tropicalis* | + | + | + | profil D |
| *C. krusei* | - | + | - | profil C |
| *C. parapsilosis* | - | - | - | profil E' |

### 1.4.3/ - Bilan

Il ressort des résultats présentés que, sur la base de la détection de trois activités métaboliques particulières, que sont les activités :
- α-glucosidase,
- phosphatase acide et
- N-acétyl-β-glucosaminidase,
il est possible de discriminer les levures du genre *Candida,* selon l'espèce à laquelle elles appartiennent, et ce de manière relativement simple. Pour ce faire, il convient de placer les levures à identifier dans des conditions de culture particulières, en l'occurrence en les plaçant en présence de modulateurs (inducteurs et répresseurs) différentiels d'activité métabolique savamment choisis. Ce faisant, sur la base de l'expression de trois activités enzymatiques de référence seulement, une grande diversité de profils d'activité métabolique est générée, qui est relativement bien représentative de la diversité des *Candida spp* ; chaque profil d'activité métabolique ainsi identifié peut être associé à quelques espèces particulières de levure du genre *Candida.*

En appliquant ce principe aux six espèces majeures de levure du genre *Candida* (à savoir *C*. *auris, C. albicans, C. glabrata, C. tropicalis, C. krusei* et *C. parapsilosis*)*,* cinq profils d'activité métabolique spécifiques ont été établis (cf. Tableau 5 et Tableau 6). Quatre de ces profils présentent une grande spécificité, chacun étant associé à une espèce particulière de levure du genre *Candida* (à savoir, *C*. *auris, C. albicans, C. tropicalis* ou *C. parapsilosis*)*.*

S'agissant de *C*. *glabrata* et de *C*. *krusei,* ces deux espèces partagent un même profil d'activité métabolique selon l'invention ; toutes deux expriment uniquement l'activité phosphatase acide dans les conditions opératoires de la présente invention. La mise en culture de ces deux espèces sur un milieu de culture solide permet de les distinguer très facilement. En effet, sur les supports de culture solide, les levures appartenant à l'espèce *C*. *glabrata* forment classiquement des colonies à bord(s) plutôt lisse(s), alors que les levures appartenant à l'espèce *C*. *krusei* forment de colonies à bords dentelés.

Il convient aussi de noter qu'en se basant sur une détection d'un profil d'activité métabolique, combinant uniquement les activités α-glucosidase, phosphatase acide et N-acétyl-β-glucosaminidase, et en l'absence de répresseurs différentiels, il n'est pas possible d'obtenir une discrimination aussi parfaite des *Candida spp.* Il n'est pas non plus possible d'obtenir une identification de *C*. *auris,* avec une aussi bonne spécificité. Le Tableau 7 ci-après schématise très brièvement et de manière synthétique une tentative de discrimination de *Candida spp* dans des conditions opératoires très similaires à l'invention, mais en l'absence de répresseurs différentiels, tels que galactose et KH₂PO₄.

Le signe (+) indique que l'activité enzymatique d'intérêt a effectivement été constatée et repérée grâce à une coloration franche des colonies. Le signe (-) indique que des colonies sont bien apparues sur le milieu de culture mais que celles-ci sont restées incolores ; ceci atteste d'une activité enzymatique d'intérêt non détectée dans les conditions de répression testées. Le signe (+/-) indique une activité enzymatique d'intérêt quelque peu moins prononcée, repérée par une coloration moins intense des colonies.

**Tableau 7**

| | α-glucosidase (+ maltose) | phosphatase acide (+ glucose) | N-acétyl-β-glucosaminidase (+ N-acétyl-β-glucosamine) | Profil métabolique |
|---|---|---|---|---|
| *C. auris* | + | + | - | profil 1 |
| *C. albicans* | + | + | + | profil 2 |
| *C. glabrata* | - | + | - | profil 3 |
| *C. tropicalis* | + | + | + | profil 2 |
| *C. krusei* | - | + | - | profil 3 |
| *C. parapsilosis* | +/- | + | - | profil 1 /profil 1' |

### 2/ - Mise au point de milieux de culture solides à des fins de détection du profil d'activité métabolique discriminant de levures du genre Candida, pour une identification selon l'espèce

A travers la conception d'un milieu de culture solide et chromogène, les inventeurs ont trouvé une manière simple et rapide d'appliquer le principe démontré ci-dessus, et permettant une identification et discrimination visuelles des *Candida spp* sur une reconnaissance colorimétrique d'un profil d'activité métabolique particulier induit, par des conditions opératoires établies. Les inventeurs ont ainsi oeuvré à la mise au point d'un milieu de culture solide :
- établissant les conditions de culture appropriées aux levures à étudier et propices à orienter l'activité métabolique desdites levures étudiées vers des profils d'activité métabolique d'intérêt bien établis, et
- permettant une lecture directe des résultats, à savoir une identification des espèces de levure se développant à la surface dudit milieu de culture solide, en se basant sur la couleur des colonies formées, éventuellement également sur leur morphologie.

Dans ce contexte, les inventeurs ont mis au point et testé différents milieux de culture solides, en faisant varier notamment :
- la composition nutritive de base,
- la nature et la concentration finale dans le milieu, des inducteurs et répresseurs d'activité métabolique,
- les substrats chromogènes pour chacune des activité enzymatiques d'intérêt.

Pour chaque milieu de culture élaboré, les tests ont été effectués en y ensemençant 10 µL d'une suspension de levures concentrée à 10⁶ CFU/mL. Une fois ensemencés, les milieux de culture ont été incubés à 35°C (± 2°C). La lecture des résultats a été effectuée après 24 heures (± 4 heures) ou après 48 heures (± 6 heures) d'incubation. Les performances de culture ont alors été analysées, en termes de densité et de taille des colonies, ainsi que de teinte et d'intensité de coloration de ces colonies et, éventuellement, en termes de morphologie des colonies.

La description ci-après reprend en partie les résultats obtenus et correspondent à des données spécifiquement sélectionnées dans l'objectif de faciliter la compréhension quant à l'élaboration de milieux de culture solides chromogènes selon l'invention.

### 2.1 / - Milieu de culture solide et composante nutritive de base

Pour réaliser un milieu de culture solide selon l'invention, il est possible de partir d'un grand nombre de compositions comprenant une composante nutritive de base adaptée à la culture des levures du genre *Candida.*

Les exemples qui vont suivre font référence à deux milieux de culture de composition relativement basique, adaptée à la culture des levures du genre *Candida,* et à partir desquels, il suffit d'ajouter quelques ingrédients additionnels appropriés (tels que des substrats chromogènes et/ou fluorogènes, des inducteurs et des répresseurs d'activité métabolique) pour obtenir des milieux de culture solides selon l'invention.

Le Tableau 8 ci-après détaille la composition de ces deux milieux, notés Milieu 1 et Milieu 2.

**Tableau 8**

| | | Concentration (g/L) | % m/V |
|---|---|---|---|
| Milieu 1 | | | |
| | Mélange d'agars | 14 | 1,4 |
| | Extrait de levure | 6 | 0,6 |
| | Extrait de malt | 4,5 | 0,45 |
| | Glucose | 1 | 0,1 |
| | Chlorure de manganèse, 4H₂O | 0,015 | 0,0015 |
| | N-acétyl-β-D-glucosaminide | 1 | 0,1 |

| Milieu 2 | | | |
|---|---|---|---|
| | Mélange d'agars | 14 | 1,4 |
| | Peptone de gélatine | 5 | 0,5 |
| | Hydrolysat de caséine | 5 | 0,5 |
| | Glucose | 1 | 0,1 |
| | Chlorure de manganèse, 4H₂O | 0,015 | 0,0015 |
| | N-acétyl-β-D-glucosaminide | 1 | 0,1 |

Ces deux milieux, Milieu 1 et Milieu 2, diffèrent l'un et l'autre par leur composante nutritive (un mélange d'extrait de levure et d'extrait de malt pour l'un, et un mélange de peptones de gélatine et de caséine, pour l'autre).

Bien entendu, d'autres composantes nutritives adaptées à la culture de levures existes et peuvent très bien être envisagées pour la mise en oeuvre de la présente invention (par exemple d'autres mélanges d'extrait de levure et/ou de peptones et/ou d'hydrolysat de caséine et/ou d'hydrolysat pancréatique et/ou d'extrait de viande ; *cf.* par exemple les quelques compositions des milieux de culture pour levures du genre *Candida,* telles que listées dans l'ouvrage « Handbook of Microbiological Media - Fourth Editi*on »*)*.*

### 2.2/ - Sélection des substrats chromogènes

Pour marquer visuellement les activités enzymatiques cibles exprimées par les levures mises en culture sur le milieu de culture solide, nombre de substrats chromogènes sont disponibles sur le marché, et offrent une riche palette de couleurs. A cet égard et à titre d'exemple, on peut citer les substrats chromogènes de la famille indoxyl, parmi laquelle :
- les chromogènes porteurs d'un groupe 5-bromo-4-chloro-3-indolyl libèrent un chromophore de teinte bleue,
- ceux porteurs d'un groupe 3-indolyl libèrent également un chromophore de teinte bleue,
- ceux porteurs d'un groupe 5-bromo-6-chloro-3-indolyl libèrent un chromophore de teinte rouge magenta,
- ceux porteurs d'un groupe 6-chloro-3-indolyl libèrent un chromophore de teinte saumon,
- ceux porteurs d'un groupe 5-iodo-3-indoxyl libèrent un chromophore de teinte violette,
- ceux porteurs d'un groupe 5-bromo-4-chloro-3-indolyl-N-méthyl libèrent un chromophore de teinte verte.

Ces chromogènes se déclinent en divers substrats chromogènes spécifiques d'une activité enzymatique particulière, et notamment en :
- substrats chromogènes d'a-glucosidase, comme par exemple le 5-bromo-4-chloro-3-indolyl-α-glucoside (ou X-α-Glu),
- substrats chromogènes de phosphatase, comme par exemple le 6-chloro-3-indolyl-phosphate (ou Rose-Phos ou Sal-Phos), et
- substrats chromogènes de N-acétyl-β-glucosaminidase, comme par exemple le 5-bromo-6-chloro-3-indolyl-β-N-acétyl-glucosaminide (ou Mag-NAG).

A côté des substrats chromogènes du type indoxyl, bien d'autres familles existent et sont disponibles dans le commerce, par exemple des substrats porteur d'un noyau coumarine, d'un noyau hydroxyquinoline, les substrats dérivés d'indoxyl à base d'Aldol^{®} de la société suisse BIOSYNTH AG, et dans lesquelles il est envisageable de piocher les substrats d'α-glucosidase, de phosphatase et de N-acétyl-β-glucosaminidase en vue de mettre en oeuvre la présente invention.

Pour chacune des trois activités métaboliques d'intérêt, les inventeurs ont sélectionné et évalué différents substrats chromogènes en vue d'identifier les plus appropriées pour une utilisation dans des milieux de culture solides, et dont l'utilisation en combinaison avec d'autres substrats sélectionnés pouvait offrir un contraste satisfaisant pour une bonne discrimination des profils d'activité métabolique induits selon l'invention.

Également, les inventeurs ont étudié les interactions éventuelles entre les substrats chromogènes sélectionnés et les éléments nutritifs qui entrent dans la composition du support solide de culture.

### 2.2.1/ - Substrats chromogènes d'α-glucosidase

Pour l'activité α-glucosidase, des substrats chromogènes tels que le 5-bromo-4-chloro-3-indolyl-α-glucoside (ou X-α-Glu), l'Aldol^{®} 455-α-glucoside (ou Aldol^{®} 455-α-Glu), l'Aldol^{®} 484-α-glucoside (Aldol^{®} 484-α-Glu) et l'Alizarine-α-glucoside, ont été évaluée.

Les essais réalisés, par exemple, avec *C*. *auris* ont abouti aux constats suivants, après 24 heures de culture :
- le 5-bromo-4-chloro-3-indolyl-alpha-glucoside (ou X-α-Glu), utilisé par exemple à une concentration de 0,1 g/L, teint en bleu les colonies des souches de clades I, III et IV, et en un bleu moins intense des colonies des souches de clades II ; il convient de noter que la teinte des colonies peuvent tirer davantage vers le vert lorsque que le milieu est plus fortement acide,
- l'Aldol^{®} 455-alpha-glucoside (ou Aldol^{®} 455-α-Glu), utilisé par exemple à 0,1 g/L, colore en j aune les colonies de clades I, III and IV ; mais cette coloration n'est pas visible avec les colonies isolées de clade II, de faible densité ;
- l'Aldol^{®} 484-alpha-glucoside (Aldol^{®} 484-α-Glu), utilisé à une concentration de 0,1 g/L, donne aux colonies une coloration orange de faible intensité et ce, quelle que soit la clade ;
- l'Alizarine-α-glucoside, par exemple utilisé à 0,05 g/L en combinaison avec du citrate de fer à 0,05 g/L, colore en violet les colonies de clades I, III and IV, et en violet plus clair les colonies de clade II.

### 2.2.2/ - Substrats chromogènes de phosphatase, et induction d'une activité phosphatase acide.

Les phosphatases acides étant caractérisées par un pH d'activité optimale acide, leur mise en évidence nécessite de placer les levures dans un environnement acide. Pour ce faire, les milieux de culture sont complémentés avec un sucre fermentaire (par exemple le glucose) et un système tampon adapté pour maintenir le milieu à un pH de l'ordre de 5,5 à 6,5 (par exemple un tampon phosphate). La fermentation de ce sucre par les levures libère localement des entités acides, qui profitent aux levures en stimulant leur activité phosphatase acido-dépendante.

L'éventuelle activité phosphatase (aussi bien acide qu'alcaline) exprimée par les levures du genre *Candida* a été mise en évidence en utilisant des substrats chromogènes de phosphatase. A cet effet, différents substrats chromogènes sont disponibles sur le marché. Par exemple, ceux dont l'hydrolyse libère un noyau chromophore 6-chloro-3-indolyl donnent une teinte rose aux colonies et ceux libérant un noyau 5-bromo-6-chloro-3-indolyl donne une teinte magenta.

Également, l'influence d'un sel inorganique de phosphate sur cette activité phosphatase acide a-t-elle aussi été évaluée.

Chaque souche de levure testée a ainsi été cultivée sur un milieu gélosé solide (notamment de composition telle que Milieu 1 ou Milieu 2) tamponné et contenant, entre autres :
- du glucose à différentes concentrations finales dans le milieu, en l'occurrence, une concentration finale inférieure à 20 g/L, notamment de 1 g/L à 20 g/L, de préférence de 5 g/L ;
- du KH₂PO₄ à différentes concentrations finales dans le milieu, en l'occurrence à des concentrations finales allant de 0,5 g/L à 3 g/L, de préférence 2 g/L ;
- un substrat chromogène spécifique aux phosphatases (comme le 5-bromo-6-chloro-3-indolyl-phosphate (Mag-Phos), l'Aldol^{®} 470-phosphate, l'Aldol^{®} 495-phosphate, l'Aldol^{®} 515-phosphate).

Après 48 heures de culture à 35°C, il a pu être constaté qu'avec la plupart des substrats chromogènes (à l'exception du substrat Aldol^{®} 515-phosphate) :
- *chez C. auris, C. albicans* et *C. parapsilosis,* l'activité phosphatase s'exprime, elle ne semble pas être dépendante du glucose et peut être réprimée avec l'addition de KH₂PO₄ ;
- *C*. *glabrata* exprime une activité phosphatase acide seulement en présence de glucose et ceci, quelles que soient les concentrations en KH₂PO₄ du milieu, enfin
- chez *C*. *tropicalis* et *C*. *krusei,* l'activité phosphatase s'exprime de manière soutenue et ne semble pas être dépendante du glucose ; elle ne semble pas non plus être réprimée par le KH₂PO₄.

### 2.2.3/ - Substrats chromogènes de la N-acétyl-β-glucosaminidase

Pour l'activité N-acétyl-β-glucosaminidase, différents substrats chromogènes ont été testés. Aucun comportement particulier n'a été constaté avec les substrats testés.

### 2.3 / - Exemples de quelques incidences possibles de la composante nutritive sur les profils d'activité métabolique induits selon l'invention

### 2.3.1/ - Incidence des extraits de levure

Dans le Tableau 9 ci-après, sont compilés quelques conditions opératoires particulières faisant apparaître d'éventuels impacts que peut avoir certaines composantes nutritives du milieu de culture, en particulier les composantes nutritives renfermant des extraits de levure, sur la détection des activités métaboliques d'intérêt selon l'invention.

**Tableau 9**

| | | Milieu 1 | | | Milieu 2 | |
|---|---|---|---|---|---|---|
| | incubation | | + X-NAG | + X-NAG | + X-NAG | + X-NAG |
| | | + X-NAG | + Mag-Phos | + Mag-Phos | + Mag-Phos | + Mag-Phos |
| | | + Mag-Phos | + glucose (7 g/L) | + glucose (20 g/L) | + glucose (20 g/L) | + glucose (6 g/L) |
| | | | + KH₂PO₄ (0,5 g/L) | + KH₂PO₄ (0,5 g/L) | + KH₂PO₄ (0,5 g/L) | + KH₂PO₄ (2 g/L) |
| *C. auris* | 48 h | rose | rose | incolore | rose | rose très pâle |
| *C. albicans* | 24 h | bleu | bleu clair | vert pâle | incolore | incolore |
| | 48 h | bleu | vert | vert | vert | bleu |
| *C. glabrata* | 48 h | incolore | incolore | incolore | rose | rose |
| *C. tropicalis* | 48 h | violet | violet | violet | violet | violet |
| *C. krusei* | 48 h | rose | rose | rose | rose | rose |
| *C. parapsilosis* | 48 h | incolore | incolore | incolore | rose clair | rose très pâle |
| | pH | 6,5 | 6,2 | 6 à 6,2 | 6 | 5,8 |

Avec le Milieu 1, une absence d'expression d'activité phosphatase acide est constaté chez *C*. *glabrata.* Il a pu être montré que l'extrait de levure présente dans la composante nutritive de ce milieu agit comme un inhibiteur sur l'activité phosphatase acide de cette espèce (cf. Tableau 10 ci-après).

L'extrait de levure de ce milieu, combiné à une forte concentration de glucose (20 g/L), inhibe également l'activité phosphatase acide chez *C. auris* (agissant ainsi compétitivement ou additionnellement avec KH₂PO₄).

Avec le Milieu 2, exempte de tout extrait de levure, ce phénomène d'inhibition de l'activité phosphatase acide chez *C*. *glabrata* et *C*. *auris* n'est plus observée.

**Tableau 10**

| | | Milieu 2 | Milieu 2 + extrait de levure (0,5 g/L) | Milieu 2 + extrait de levure (2 g/L) |
|---|---|---|---|---|
| | incubation | + X-NAG | + X-NAG | + X-NAG |
| | | + Mag-Phos | + Mag-Phos | + Mag-Phos |
| | | + glucose (6 g/L) | + glucose (6 g/L) | + glucose (6 g/L) |
| | | + KH₂PO₄ (2 g/L) | + KH₂PO₄ (2 g/L) | + KH₂PO₄ (2 g/L) |
| *C. auris* | 48 h | rose très pâle | rose très pâle | rose très pâle |
| *C. albicans* | 24 h | incolore | incolore | incolore |
| | 48 h | bleu | bleu | bleu |
| *C. glabrata* | 48 h | rose | rose clair | incolore |
| *C. tropicalis* | 48 h | violet | violet | violet |
| *C. krusei* | 48 h | rose | rose | rose |
| *C. parapsilosis* | 48 h | rose très pâle | rose très pâle | rose très pâle |
| | pH | 5,8 | 5,8 | 5,8 |

### 2.3.2/ - Mise en oeuvre de l'invention avec le Milieu 2

Les Tableaux 11 et 12 ci-après illustrent l'implémentation, dans le Milieu 2, des conditions de culture appropriées pour induire, chez les levures du genre *Candida* (avec ou sans galactose), une expression différentielle particulière des trois activités enzymatiques d'intérêt (α-glucosidase, phosphatase acide et N-acétyl-β-glucosaminidase) et rendant possible une discrimination visuelle de *C. auris, C. albicans, C. glabrata, C. tropicalis, C. parapsilosis* et *C*. *krusei.*

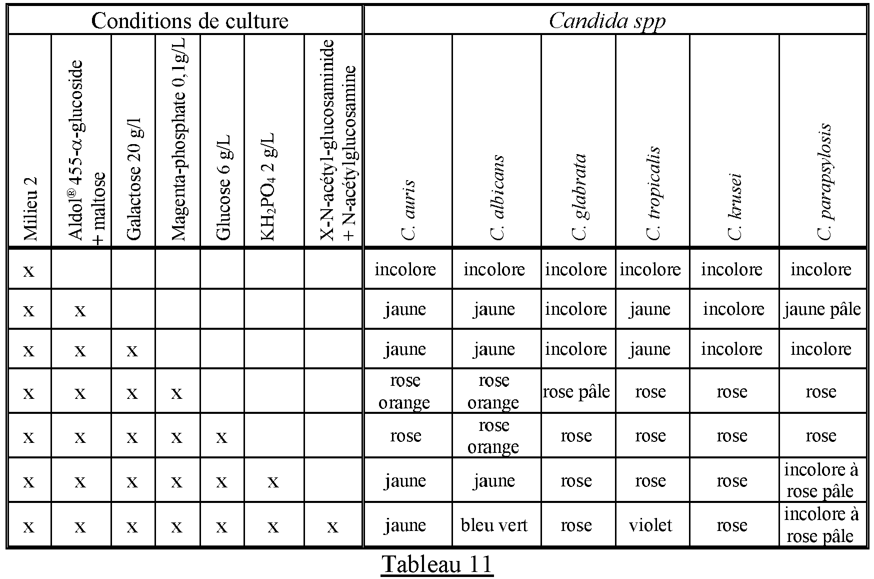

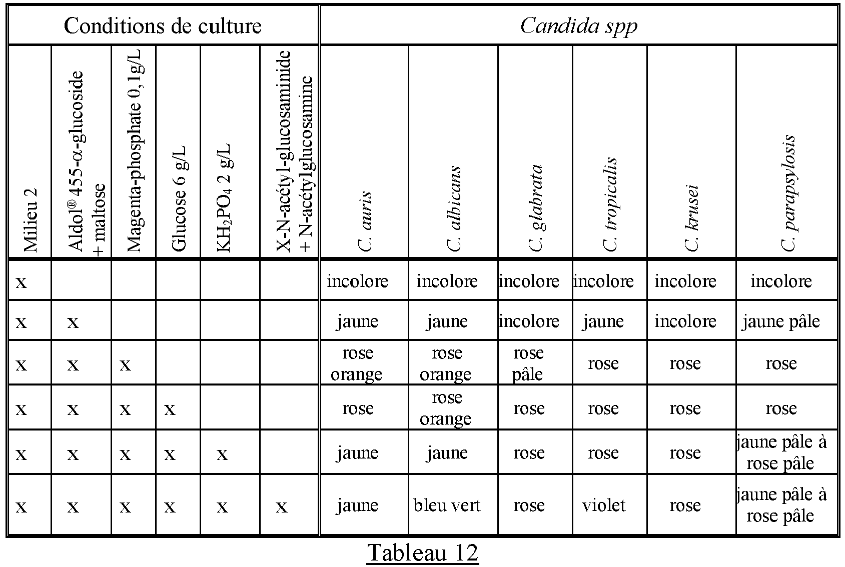

### 3/ - Optimisation de la composante nutritive d'une composition de milieu de culture solide selon l'invention

Comme précédemment exposé, la composition du Milieu 2 correspond à un mode de réalisation particulier d'une composante nutritive d'un milieu de culture solide selon l'invention. Celle-ci a démontré des résultats particulièrement intéressantes et parfaitement acceptables en termes de fertilité à l'égard des levures du genre *Candida* et, plus particulièrement, de *C. auris, C. albicans, C. glabrata, C. tropicalis, C. parapsilosis* et *C. krusei.*

Comme indiqué plus haut, d'autres compositions de composante nutritive propices au développement et la croissance des *Candida spp,* peuvent être envisagées pour mettre en oeuvre la présente invention. Ces autres compositions de composante nutritive font partie de l'état de la technique, voire de la connaissance générale de l'homme du métier, notamment elles peuvent se retrouver dans le manuel « Handbook of Microbiological Media - Fourth Editi*on* » ou en découler.

Néanmoins, comme vu précédemment avec le Milieu 1, les composantes nutritives contentant des extraits levure sont à éviter. Ainsi, pour la préparation d'un milieu de culture solide selon l'invention. Il est conseillé d'utiliser les composantes nutritives pour *Candida spp,* qui soient à faible teneur en extrait de levure (voire totalement exemptes d'extrait de levure) ou bien alors prévoir une substitution, au moins partielle, des extraits de levure par des alternatives nutritionnelles, plus ou moins complexes, telles que par exemple des extraits de viande, des peptones, des extrait de malt...

En partant de la composition du Milieu 2 (cf. Tableau 8), les inventeurs ont investigué différents points d'amélioration, différentes approches, en vue d'en optimiser les performances, notamment la fertilité. A cet égard, des travaux complémentaires ont porté, entre autres, sur :
- l'évaluation de diverses peptones provenant de différents fournisseurs et, en particuliers, des alternatifs à la peptone de gélatine, tels que la peptone de pomme de terre, la peptone de lupin, la bioSoyase (une peptone papaïnique de soja), des extraits de malt, de caséine ;
- le remplacement du glucose par le pyruvate de sodium ;
- l'optimisation du pH acide du milieu, et le système tampon approprié.

Le tableau 13 ci-après donne un exemple d'optimisation du Milieu 2.

**Tableau 13**

| | Concentration (g/L) | % m/V |
|---|---|---|
| Mélange d'agars | 14 | 1,4 |
| Peptone de pomme de terre | 5 | 0,5 |
| Hydrolysat de caséine | 5 | 0,5 |
| Extrait de malt | 3 | 0,3 |
| Glucose | 1 | 0,1 |
| Chlorure de manganèse, 4H₂O | 0,015 | 0,0015 |
| N-acétyl-β-D-glucosamine | 1 | 0,1 |
| Hydrogénophosphate de dipotassium/phosphate de potassium dibasique (K₂HPO₄) | 0,5 | 0,05 |
| pH | 6,2 ± 0,3 | |

## Revendications

1. Milieu de culture microbiologique solide, pour la détection et l'identification de levures du genre *Candida* susceptibles d'être présentes dans un échantillon biologique à tester, ledit milieu comprenant :
- une composante nutritive apte à permettre le développement et la croissance de levures du genre *Candida,* et
- une composante discriminante apte à permettre un marquage visuel de colonies de levures poussant sur ledit milieu de culture, et
étant **caractérisé en ce que** ladite composante discriminante comprend :
- un inducteur d'a-glucosidase et un substrat chromogène et/ou fluorogène d'a-glucosidase qui, sous l'action d'une α-glucosidase, libère un chromophore et/ou un fluorophore émettant un premier signal optique détectable,
- un inducteur de phosphatase acide, un répresseur de phosphatase et un substrat chromogène et/ou fluorogène de phosphatase qui, sous l'action d'une phosphatase, libère un chromophore et/ou un fluorophore émettant un deuxième signal optique détectable, ledit deuxième signal optique détectable étant différent dudit premier signal optique détectable,
- un inducteur de N-acétyl-β-glucosaminidase et un substrat chromogène et/ou fluorogène de N-acétyl-β-glucosaminidase qui, sous l'action d'une N-acétyl-β-glucosaminidase, libère un chromophore et/ou un fluorophore émettant un troisième signal optique détectable, ledit troisième signal optique détectable étant différent dudit premier signal optique détectable et dudit deuxième signal optique détectable.

2. Milieu de culture selon la revendication 1, lequel comprend à titre d'inducteur d'α-glucosidase, un sucre choisi parmi le maltose et le raffinose, à une concentration comprise entre 0,05 g/L et 0,5 g/L.

3. Milieu de culture selon la revendication 1 ou 2, lequel comprend à titre d'inducteur de phosphatase acide, un sucre fermentaire métabolisable par les levures et un tampon pH apte à maintenir ledit milieu à un pH acide.

4. Milieu de culture selon la revendication 3, lequel comprend un tampon pH maintenant le pH entre 5,5 et 6,5.

5. Milieu de culture selon la revendication 3 ou 4, lequel comprend, à titre de sucre fermentaire, du glucose à une concentration comprise entre 5 g/L et 20 g/L.

6. Milieu de culture selon l'une quelconque des revendications 1 à 5, lequel comprend, à titre de répresseur de phosphatase, un sel inorganique de phosphate, de préférence du phosphate de potassium à une concentration comprise entre 0,5 g/L et 3 g/L, de préférence 2 g/L.

7. Milieu de culture selon l'une quelconque des revendications 1 à 6, lequel comprend, à titre d'inducteur de N-acétyl-β-glucosaminidase, un hexosamine, de préférence le N-acétyl-β-glucosamine.

8. Milieu de culture selon l'une quelconque des revendications 1 à 7, dans lequel ledit substrat d'a-glucosidase est un substrat chromogène porteur d'un noyau indoxyl (1H-indolyl-3-yl) conjugué sur l'amine cyclique.

9. Milieu de culture selon l'une quelconque des revendications 1 à 8, lequel comprend un répresseur d'α-glucosidase, choisi entre le galactose et le xylose, à une concentration comprise entre 5 g/L et 30 g/L.

10. Milieu de culture selon l'une quelconque des revendications 1 à 9, dans lequel la composante nutritive est exempte d'extrait de levure.

11. Procédé d'identification de levures du genre *Candida* éventuellement présentes dans un échantillon biologique, comprenant les étapes suivantes :
- mise en culture des levures à identifier sur ou dans un milieu de culture comprenant :
- une composante nutritive apte à permettre le développement et la croissance de levures du genre *Candida,*
- un inducteur d'α-glucosidase,
- un inducteur de phosphatase acide et un répresseur de phosphatase, et
- un inducteur de N-acétyl-β-glucosaminidase,
- détection d'éventuelles activités α-glucosidase, phosphatase acide et/ou N-acétyl-β-glucosaminidase exprimées par les levures, et
- identification de l'espèce à laquelle lesdites levures appartiennent selon les activités α-glucosidase, phosphatase acide et/ou N-acétyl-β-glucosaminidase effectivement exprimées par lesdites levures.

12. Procédé d'identification selon la revendication 11, dans lequel la détection desdites activités α-glucosidase, phosphatase acide et N-acétyl-β-glucosaminidase est réalisée en utilisant des substrats chromogènes et/ou fluorogènes.

13. Procédé d'identification selon la revendication 11 ou 12, dans lequel la détection desdites activités α-glucosidase, phosphatase acide et/ou N-acétyl-β-glucosaminidase est réalisée en utilisant un milieu de culture microbiologique solide tel que défini par l'une quelconque des revendications 1 à 10.

14. Procédé de détection et/ou d'identification de *C*. *auris* éventuellement présentes dans un échantillon biologique, comprenant les étapes suivantes :
- mise en culture des éventuelles levures à détecter et/ou à identifier sur ou dans un milieu de culture comprenant :
- une composante nutritive apte à permettre le développement et la croissance de levures du genre *Candida,*
- un inducteur d'α-glucosidase, ,
- un inducteur de phosphatase acide et un répresseur de phosphatase, et
- un inducteur de N-acétyl-β-glucosaminidase,
- détection d'éventuelles activités α-glucosidase, phosphatase acide et/ou N-acétyl-β-glucosaminidase exprimées par les levures, et
- identification comme *C*. *auris,* les levures qui expriment une activité α-glucosidase et n'expriment ni une activité phosphatase acide, ni une activité N-acétyl-β-glucosaminidase.

15. Procédé de détection et/ou d'identification selon la revendication 14, dans lequel lesdites activités α-glucosidase, phosphatase acide et N-acétyl-β-glucosaminidase sont détectées en utilisant des substrats chromogènes et/ou fluorogènes.

16. Procédé de détection et/ou d'identification selon la revendication 14 ou 15, dans lequel la détection desdites activités α-glucosidase, phosphatase acide et N-acétyl-β-glucosaminidase est réalisée en utilisant un milieu de culture microbiologique solide tel que défini par l'une quelconque des revendications 1 à 10.
